# EUROPEAN PATENT SPECIFICATION

(11) **EP 1 025 841 B1**
(45) Date of publication and mention of the grant of the patent: **05.06.2002**
(21) Application number: 99204294.5
(22) Date of filing: 31.01.1995
(51) Int. Cl.: A61K 9/20, A61K 31/43

(54) **Bilayered amoxycillin tablets**
Zweischichtige Amoxycillintabletten
Tablettes d'amoxycilline bicouches

(30) Priority: 04.02.1994 GB 9402203
(43) Date of publication of application: 09.08.2000
(62) Divisional of application: 95908234.8
(73) Proprietor: SMITHKLINE BEECHAM PLC, Brentford, Middlesex TW8 9EP (GB)
(72) Inventor: Ebbers, Walter F. W., Jago Pharma AG, 4132 Muttenz (CH); Zimmer, Robert H., Jago Pharma AG, 4132 Muttenz (CH)
(74) Representative: Connell, Anthony Christopher

(56) References cited:
- WO-A-94/06416
- WO-A-94/27557
- WO-A-95/28148

## Description

This invention relates to novel tablet formulations for oral administration of amoxycillin optionally together with clavulanic acid.

Amoxycillin and clavulanic acid are respectively a known β-lactam antibiotic and a known β-lactamase inhibitor. Their use together in antibiotic formulations is disclosed in for example GB 2005538, which discloses formulations comprising amoxycillin trihydrate and potassium clavulanate. In oral formulations amoxycillin is generally used in the form of its trihydrate, and clavulanic acid is generally used in the form of a pharmaceutically acceptable salt (hereinafter "clavulanate") in particular potassium clavulanate.

It is desirable to provide oral pharmaceutical formulations which release their active material content at a controlled rate after oral administration, so that for example release of the active material into the stomach or intestine occurs over a period of several hours after ingestion of the formulation. This can allow unit doses of the formulation to be taken at widely separated intervals, for example twice daily, whilst maintaining a therapeutically effective plasma level of the active material content, such as amoxycillin and clavulanate.

Among various ways of achieving a controlled rate of release are layered tablets. For example GB 1346609 discloses three layer tablets having two drug-containing layers located one on each side of an inert disintegrable intermediate layer. The only drug substance mentioned in GB 1346609 is Mepyrizole. US 4122157 discloses a controlled release nitrofurantoin tablet which comprises two adjacent layers, one being a rapid release layer, the other being a slow release layer. US 4,839,177 discloses controlled release tablets comprising a deposit-core containing an active drug substance, with a support platform applied to this deposit core.

WO 94/27557 (SmithKline Beecham) describes a process for preparing controlled release solid dosage forms of pharmaceutically active materials which comprises the thermal infusion of a pharmaceutically active material and a hydrophobic waxy material into thermal infusion granules. Examples are provided therein of bilayer tablets in which one of the layers contains amoxycillin. This application was published on 8 December 1994, after the priority date of the present application.

WO 94/06416 (Jagotec AG) describes bilayer tablets in which the first layer has an immediate or controlled release formulation and the second layer has a slow release formulation, modified by the presence of a further low-permeability barrier-type layer surrounding the second layer or placed between the first and second layers. An example is provided therein of a tablet comprising 500mg of amoxycillin trihydrate, distributed equally between an immediate release layer and a slow release layer, with a barrier layer to further modify release from the slow release layer. This application was published on 31 March 1994, after the priority date of the present application.

Particular problems are encountered with making controlled-release formulations which include clavulanate in combination with antibiotics such as amoxycillin. Amoxycillin trihydrate and clavulanate differ substantially in their solubility in water. Potassium clavulanate is very water soluble and amoxycillin trihydrate is relatively insoluble, so it is difficult to avoid rapid leaching out of potassium clavulanate from formulations containing these two in admixture. Also clavulanate is relatively sensitive to hydrolysis, so degradation of clavulanate may occur on contact with aqueous gastrointestinal contents during the relatively long periods between oral administration of controlled-release formulations.

According to this invention there is provided a bilayer tablet formulation for oral administration, consisting of a first layer which includes amoxycillin and a second layer which includes amoxycillin wherein the relative rate of release of amoxycillin and/or clavulanate from the first and second layers differs and optionally a coating layer.

Amoxycillin may suitably be used in the form of amoxycillin trihydrate, and clavulanate may be used in the form of potassium clavulanate. The tablet formulation may contain other antibacterial agents in addition to amoxycillin and clavulanate. Amoxycillin, clavulanate and any such other antibacterial agents which may be present are herein singly and collectively referred to as "active material content" unless otherwise specifically identified.

Tablet formulations of this invention may provide extended plasma levels of their active material content, for example of amoxycillin and clavulanate after ingestion.

When the formulation of the invention contains amoxycillin and clavulanate the overall weight ratio of amoxycillin : clavulanate (expressed as the free acid equivalent) may vary between broad limits, for example between 30 : 1 to 1 : 1. Suitably the ratio may be between 8 : 1 to 1 : 1, for example between 7 : 1 to 1 : 1, typically around 4 : 1, eg between 3.5 : 1 and 4.5 : 1. When both of the layers comprise amoxycillin and clavulanate the amoxycillin: clavulanate ratio in each layer may be the same as the overall tablet ratio or there may be different ratios in each of the layers, making up the ratio in the overall tablet. For example one layer may contain amoxycillin without clavulanate and the other layer may contain amoxycillin plus clavulanate, making up the ratio in the overall tablet.

Suitably the tablet formulations of the invention may contain up to the maximum permitted daily dose of amoxycillin and clavulanate per unit dose. The formulations may for example contain nominally around 875 mg of amoxycillin and around 250 mg of clavulanate. Suitably unit dose tablets of the invention may contain the following nominal weights (mg) of amoxycillin: clavulanate (expressed as free acid equivalent); 875 : 125, 500 : 250, 500 : 125; 250 : 125 and 250 : 62.5.

The differing relative rate of release of active material content from the first and second layers of the tablet may be achieved in various ways.

For example differing rates of release may be achieved by a first layer which is a rapid release layer, ie which releases the bulk of its active material content within a relatively short time, for example within 1 hour, eg within 30 minutes after oral injestion, and a second layer which is a slow release layer, ie which releases the bulk of its active material content during a relatively long period after oral ingestion or is a delayed release layer which releases the bulk of its active material content after a period of delay after oral ingestion, either in the stomach or in the intestine.

Rapid release layers may for example be rapid disintegrating layers having a composition similar to that of known rapid-disintegrating tablets. For example, the composition may comprise principally active material content, binders such as plasdone K29-32 (trade mark), compression aids, fillers or diluents such as mannitol, microcrystalline cellulose, and silicon dioxide, eg Syloid (trade mark), disintegrants, eg Explotab (trade mark), Avicel (trade mark) lubricants such as talc, magnesium stearate etc. Suitably such a rapid release layer may comprise around 30 - 70 % (all percentages given herein are on a weight percentage basis unless otherwise stated) eg 50-60% of active material content, around 10 - 30% of fillers/compression aids, and conventional amounts of disintegrants and lubricants etc.

An alternative type of rapid-release layer may be a swellable layer having a composition which incorporates polymeric materials which swell rapidly and extensively in contact with water or aqueous media, to form a water permeable but relatively large swollen mass. Active material content may be rapidly leached out of this mass.

Slow release layers may have a composition which comprises active material content together with a release retarding material. Suitable release retarding materials include pH sensitive polymers, e.g. the known Eudragit (trade mark) polymers, for example Eudragit L (trade mark), i.e polymers based upon methacrylic acid copolymers, used either alone or with a plasticiser, release-retarding polymers which have a high degree of swelling in contact with water or aqueous media such as the stomach contents, polymeric materials which form a gel on contact with water or aqueous media, and polymeric materials which have both swelling and gelling characteristics in contact with water or aqueous media.

Polymers which have a high degree of swelling include, inter alia, cross-linked sodium carboxymethylcellulose, cross-linked hydroxypropylcellulose, high-molecular weight hydroxypropylmethylcellulose, carboxymethylamide, potassium methacrylatedivinylbenzene co-polymer, polymethylmethacrylate, cross-linked polyvinylpyrrolidone, high-molecular weight polyvinylalcohols etc. Gellable polymers include methylcellulose, carboxymethylcellulose, low-molecular weight hydroxypropylmethylcellulose, low-molecular weight polyvinylalcohols, polyoxyethyleneglycols, non-cross linked polyvinylpyrrolidone etc. Polymers simultaneously possessing swelling and gelling properties include medium-viscosity hydroxypropylmethylcellulose and medium-viscosity polyvinylalcohols.

Examples of such polymers include Methocel K4M (Trade Mark), Methocel E5 (Trade Mark), Methocel E50 (Trade Mark), Methocel E4M (Trade Mark), Methocel K15M (Trade Mark) and Methocel K100M (Trade Mark). An example of a suitable polymer mixture is a mixture of Methocel E5 and K4M, for example1:1, w:w.

Other known release-retarding polymers which may be incorporated include hydrocolloids such as natural or synthetic gums, cellulose derivatives other than those listed above, proteinaceous substances such as acacia, gum tragacanth, locust bean gum, guar gum, agar, pectin, carageenam, soluble and insoluble alginates, carboxypolymethylene, gelatin, casein, zein, Veegum (trade mark) and the like.

Such a slow release layer may contain polymers which rapidly swell in contact with water or aqueous media so that they form a relatively large swollen mass which is not rapidly discharged from the stomach into the intestine.

Suitably such a slow release layer may contain around 30-70% eg 40 - 60%, of active material content, around 15 - 45% of release-retarding polymers, around 0-30% of fillers/compression aids, conventional quantities of disintegrants and lubricants, and some 5 - 20% of soluble excipients.

Alternative types of slow- or delayed- release layer are those in which the active material content is mixed with, coated with, or embedded in a matrix of a poorly soluble or practically insoluble excipient (e.g. calcium phosphate etc) and/or a hydrophobic excipient (e.g. fats or waxes). Poorly soluble or practically insoluble excipients can yield a very hard tablet which would slowly dissolve or erode from the surface without substantial diffusion of surrounding liquid into the body of the tablet. Hydrophobic excipients similarly hinder moisture ingress and release of active material content occurs through erosion and enzymatic digestion. Examples of such waxes include Compritol (trade mark), for example Compritol HD5 and Compritol 888 (trade marks). Compritol wax remains intact in aqueous solutions at all pH, but is digested by lipase enzymes at pH 5-7. Thus active material content such as clavulanate protected with Compritol wax as described above may be released primarily in the small intestine due to the action of intestinal lipase. Typically when the slow- or delayed- release layer includes such a wax, the wax may suitably comprise 10-80% of the layer, e.g. around 20-40%, e.g. 30% ± 5% by weight.

Delayed-release layers may use the known properties of enteric polymers to delay release of active material content until the whole or part of the tablet is discharged by the stomach into the intestine after oral ingestion. Enteric polymers are insoluble or only slightly soluble in the stomach contents, but relatively soluble in the higher pH intestinal environment. Individual particles, e.g. granules of active material content may be coated with a layer of or made up with an enteric polymer, and embedded or dispersed within a soluble or disintegrable matrix. Alternatively a delayed release layer may comprise a matrix of enteric polymer within which are dispersed granules comprising active material content, and these granules may themselves be coated with an enteric polymer layer. Such granules may include conventional granulating materials. Examples of suitable enteric polymer materials include the known Eudragit (trade mark) polymers discussed above.

Such a slow-or delayed-release layer may also include fillers/compression aids such as microcrystalline cellulose, eg Avicel (trade mark), desiccants, such as silicon dioxide, eg Syloid (trade mark), disintegrants, eg Explotab (trade mark) and polyvinyl-pyrrolidone, eg polyvidon K3O (trade mark), lubricants such as talc or magnesium stearate, pH-controlling agents such as potassium dihydrogen phosphate or substantially insoluble ion-exchange resins, and soluble excipients such as mannitol or other soluble sugars.

Differing rates of release may also be achieved by having a first layer which is a slow or delayed release layer, and a second layer which is also a slow or delayed release layer. Such first and second layers may for example differ in their composition, so that they comprise different quantities, combinations or types of release-retarding materials and/or soluble excipients etc. Additionally or alternatively such first and second layers may for example differ in the relative amounts of active material content in the first and second layers.

Due to the difference in solubility of amoxycillin and clavulanate, the former may be only slowly dissolved out of a layer, whereas the latter, unless the layer is very impervious or hydrophobic, may be dissolved out relatively rapidly. Therefore if a layer contains both amoxycillin and clavulanate it may be at the same time a slow-or delayed- release amoxycillin layer but a rapid-release clavulanate layer. For example such a layer may comprise amoxycillin trihydrate, potassium clavulanate and the erodable polymer Methocel E5 (trade mark).

In this above-described tablet having two slow or delayed release layers, one or both of the first and second layers may for example be slow release layers comprising active material together with release retarding materials as described above. Alternatively one or both of the first or second layers may be a delayed release layer using enteric polymers as described above.

The tablet formulation of the invention may be wholly or partly covered by a coating layer, which may be a protective layer to prevent ingress of moisture, or damage to the tablet, or may be an enteric coating layer, for example of the known Eudragit (trade mark) polymers described above. The coating layer may itself contair active material content, and may for example by a rapid release layer, which rapidly disintegrates in contact with water or aqueous media to release its active material content for example of amoxycillin, or amoxycillin plus clavulanate combined.

As well as active material content etc, the tablet of the invention may also include a pH modifying agent, such as a pH buffer, which may be contained in either rapid-, slow-, or delayed release layers, or in a coating around all or part of the tablet. A suitable buffer is calcium hydrogen phosphate.

One embodiment of the present invention comprises a tablet having a rapid-disintegrating first layer which contains amoxycillin and/or clavulanate as active material, and a slow-release second layer which comprises amoxycillin and/or clavulanate as active material together with one or more release retarding polymers such as Methocel E5, E50, E4M, K4M, K15M or K100M or mixtures thereof, the overall tablet containing amoxycillin or amoxycillin plus clavulanate. Suitably the first layer in such a tablet contains some 20 - 35%, eg 25 - 30% of the overall active material content.

The above-mentioned embodiment of the invention may be coated with a polymer layer, for example an enteric polymer such as the Eudragit (trade mark) polymers mentioned above, or a coating layer which contains active material content, such as a rapid-release layer which includes amoxycillin and/or clavulanate.

Suitably the tablet formulations of the invention may be formed by known compression tabletting techniques for example using a known multi-layer tabletting press. Suitably a dry densification process may be used, e.g. briquetting. Typically the active material content, pH modifiers, buffers, fillers and/or diluent, release retarding agents, disintegrants and binders, when used are mixed, then lubricants and compression aids are added. The complete mixture may then be compressed under high pressure in the tablet press. Roller compaction may be used to form granulates for compaction. Alternatively wet granulation may be used, isopropanol being a suitable solvent. A suitable wet granulating aid is Polyvidon K-30 (trade mark).

For making up layers which include a wax, e.g. Compritol (trade mark) dry compression or wet granulation may be used. Typically for wet granulation a colloidal solution or suspension of the wax in a solvent such as dichloromethane may be used. Typically the solution or suspension may be mixed with the layer ingredients and the wet cake sieved and dried. This can be repeated until the required amount of wax has been incorporated.

For applying an enteric coating to particles used to make up the layers of the tablet of the invention, typically a solution or suspension of the enteric polymer, e.g. Eudragit (Trade Mark), is mixed with the active material content, e.g. powders or granules e.g. make up as described above, and the wet cake is sieved and dried. This procedure may be repeated until the required amount of the polymer is incorporated. Alternatively the granule ingredients or the granules themselves may be coated with enteric polymer by means of a fluid bed granulator, for example spray-coating the ingredients with a solution of the polymer e.g. in isopropanol.

Clavulanic acid and its derivatives are known to be extremely water sensitive, potassium clavulanate being the most stable pharmaceutically acceptable derivative. Therefore formulations which contain derivatives of clavulanic acid, such as potassium clavulanate, should be made up in dry conditions, preferably at 30% relative humidity or less, and the ingredients of the formulation should be pre-dried where appropriate. Formulations of the invention which include derivatives of clavulanic acid should be stored in containers which are sealed against the ingress of atmospheric moisture.

The invention also provides a method for the manufacture of a tablet formulation as described above comprising the steps of forming said first and second layers, and any coating layer which may be present.

The invention further provides a formulation as described above for use as a therapeutic substance for oral administration for the treatment of bacterial infections.

The invention further provides a method of use of a formulation as described above, in the manufacture of a medicament for the treatment of bacterial infections.

The invention will now be described by way of example only with reference to the accompanying drawings, in which:
Fig. 1 shows the structure of various types of tablet formulation.
Figs. 2, 3, 4, 5 and 6 show *in vitro* and *in vivo* release characteristics from tablet 6-1.

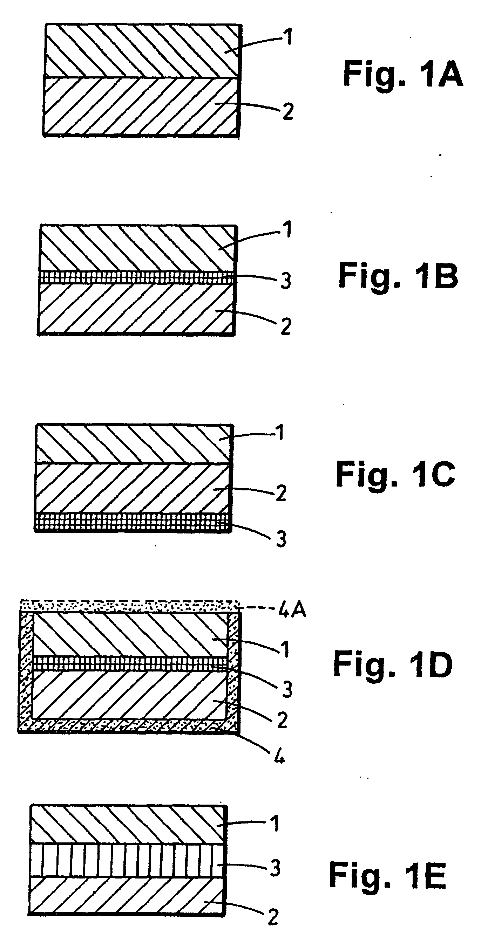

Referring to Fig 1, substantially cylindrical compressed tablets of the present invention are shown in longitudinal section. In Fig 1A, the tablet comprises a first layer (1) and a second layer (2), without any barrier layer or coating layer. In Fig 1B the tablet comprises a first layer (I), a second layer (2), and a barrier layer (3) sandwiched between the first and second layers (1) and (2). In Fig 1C, the tablet comprises a first layer (1), a second layer (2), and a barrier layer (3) located on the end face of the second layer (2). In Fig ID the tablet comprises a first layer (1), a second layer (2), a barrier layer (3) sandwiched between the first and second layers (1) and (2), and a coating layer (4) which partly covers the tablet. The dotted line shows the possibility of the coating layer (4A) covering the entire tablet. In Fig. 1E the tablet comprises a first layer (I) a second layer (2), and a third layer (3) intermediate between the first and second layers (I) and (2). All three of these layers (1), (2) and (3) include active material content.

(In the examples and figures below "AMX" is an abbreviation for amoxycillin, and "KCA" is an abbreviation for potassium clavulanate).

### Barrier Layers

In the Examples below, barrier layers L1, S2, S6 and RSB1 are referred to. The composition of these layers and the method for preparing them was as follows:

| | Amounts in % | | | |
|---|---|---|---|---|
| | **L1** | **S2** | **S6** | **RSB1** |
| Methocel K100M | 79.75% | -- | -- | -- |
| Methocel K15M | -- | -- | -- | 38.20% |
| Methocel E5 | -- | 75.50% | -- | -- |
| Methocel E50 | -- | -- | 76.50 | -- |
| Mannitol | -- | -- | -- | 38.20% |
| Hydrogenated castor oil | 13.50% | 18.80% | 18.40% | 18.50% |
| Yellow FCF aluminium lake | 0.25% | -- | -- | -- |
| Eudralak green | -- | 0.10% | 0.10% | 0.10% |
| Ethocel | 5.00% | -- | -- | -- |
| Polyvidon K30 | -- | 2.80% | 3.50% | 3.50% |
| Mg-Stearate | 1.00% | 1.90% | 1.00% | 1.00% |
| Syloid 244 | 0.50% | 0.90% | 0.50% | 0.50% |
| Total: | 100.00% | 100.00% | 100.00% | 100.00% |

The binding ingredient (Ethocel or Polyvidon K-30) was dissolved in Ethanol (94%). Methocel, Cutina HR and dye were mixed for 5 minutes in a Lödige MGT 30P at 150 r.p.m. after which the granulation solution was added. Mixing was continued for another 5 minutes. The wet granulate was dried at 45°C. The dry granulate was passed through a 1 mm sieve. Mg-stearate and Syloid 244, which had been passed through a 0.71 mm sieve, were added and the whole was mixed in the Lödige during 1 min at 150 r.p.m. to homogeneity

### Preparation of Tablets

The active ingredients, fillers and diluents (mannitol), release controlling agents (Methocels) or disintegrants (Avicel, Explotab) and binders (Plasdone K29-32) were mixed during 5 minutes in a Stefan Mixer at speed level 11. Lubricants (talc, Mg-stearate) and colloidal silicon dioxide (Syloid 244) were added, and mixing at level 1 was continued for another minute. The complete mixture was slugged on a Kilian tablet press (briquetting step), followed by size reduction (Frewitt) and passage through an oscillatory 1 mm sieve. If the flow properties were unsatisfactory, the briquetting step was repeated. The tablets of Examples 1 to 4 were prepared in this way.

The tablets of subsequent Examples were prepared by non-aqueous wet granulation. In most cases, isopropanol was chosen as the most appropriate non-aqueous solvent. In the formulations with Compritol, dichloromethane was used. In the non-aqueous granulation step an isopropanolic solution of polyvidon K-30 was mixed with a powder mixture of active ingredients and methocels (and fillers, buffers, etc.). The wet cake was pressed through a 0.5 mm sieve, the mixing was repeated and followed by sieving through a 2 mm sieve. After drying at 40°C the dry material was passed through a 1 mm sieve. Magnesium stearate and talc were added and mixed.

This granulate possessed good flow properties. In some cases, where the bulk density was rather low a densifying step (pre-tabletting and sieving as in the briquetting method) was still required in order to achieve the nominal weight of a particular layer.

Coating particles and granules with Eudragit L was performed in a similar manner. A solution of Eudragit in isopropanol was mixed with the active ingredients or with a granulate (without talc and Mg-stearate) prepared in the aforementioned wet granulation manner. The wet cake was pressed through a 0.5 mm sieve, the mixing was repeated and followed by sieving though a 2 mm sieve. After drying at 40°C the dry material was passed through a 1 mm sieve. The treatment with Eudragit, sieving and drying was repeated until the nominal amount of Eudragit L according to the recipe was contained within the granulate. Then Mg-stearate and talc were added and mixed.

Other coating work with Eudragit L involved the use of a fluid bed granulator. The active ingredients were spray-coated with an isopropanolic solution of Eudragit L in a Glatt fluid bed granulator to 20% Eudragit L of the weight of the active ingredients. Samples were taken at the 5, 10, 15 and 20% Eudragit weight level. In addition, a granulate of active ingredients with Methocels was prepared in the Glatt employing an isopropanolic solution of polyvidon K-30. This granulate was then spray-coated with Eudragit L on the Glatt.

The formulation work with Compritol either involved dry compression or a wet granulation step with a colloidal solution of Compritol in dichloromethane. The wet granulation was analogous to the Eudragit wet granulation. The treatment with the Compritol solution, sieving and drying was repeated until the nominal amount of Compritol according to the recipe was contained within the granulate. Then Mg-stearate and talc were added and mixed.

### Dissolution Testing Methods

The release of amoxycillin and clavulanate from tablets into static media was measured using the USP Dissolution Test Method II.

The release of amoxycillin and clavulanate from tablets into flowing water and other media was measured using the USP Method IV Dissolution , using large (22.6 mm, 8.3 ml/min) and small (12 mm 8.3 ml/min) cells.

The test was performed according to USP XXXII <724>, apparatus 4.

| **Dissolution apparatus:** | |
|---|---|
| Dissolution apparatus | SOTAX CE6 flow cell |
| | SOTAX CY7-50 pump |

| **Test specifications:** | |
|---|---|
| Temperature | 37.0 ± 0.5°C |
| Test medium | 0 - 2h: HCl 0.01 M (pH 2.0) 2 - 8h: phosphate buffer 0,05 M; pH = 6.6 |
| Flow rate | 8.3 ml/min (500 ml/h) |
| Flow cell | 22.6 mm diameter |

### Solutions:

- 0.05 M phosphate buffer solution 3 (pH 6.6): Dissolve 544g potassium dihydrogenphosphate in 20.0L water and adjust to pH 6.6 with NaOH 40% (m/v).
- 0.05 M phosphate buffer solution 4 (pH 6.6): Dissolve 408g potassium dihydrogenphosphate and 39g sodium hydroxide pellets p.a. in 60L water.

### Method:

Three tablets were tested each as follows. A 5 mm diameter ruby bead was placed at the bottom of the cone of the flow cell and the cone was filled with 1 mm diameter glass beads. A tablet was introduced into the cell and fixed vertically using a holder. The filter head was assembled and the parts fixed together by means of a clamping device. The dissolution medium warmed to 37.0 ± 0.5°C was introduced through the bottom of the cell at a flow rate of 8.3 ml/min. 0.01 M HCl from the beginning to 2 hours was used, then changed to buffer solution 4 until the end of the run.

The drug release after 30 min, 60 min 2 h 3 h, 4 h, 5 h, 6 h, 7 h and 8 h was determined.

As Potassium Clavulanate is very unstable at low pH values, the fractions for the time from 0 to 2 hours (medium 0.01 M HCL) were collected directly in a buffer solution and were mixed well with this buffer in a ratio of about 1:1.

### Reference Example 1

Tablets 1.1, 1.2 and 1.3, each having a structure as shown in Fig 1B were prepared using an oval biconvex punch 21 x 10 mm, having a rapid-disintegrating first layer and a slow-release second layer, with a barrier layer between. The composition of these tablets is as follows:

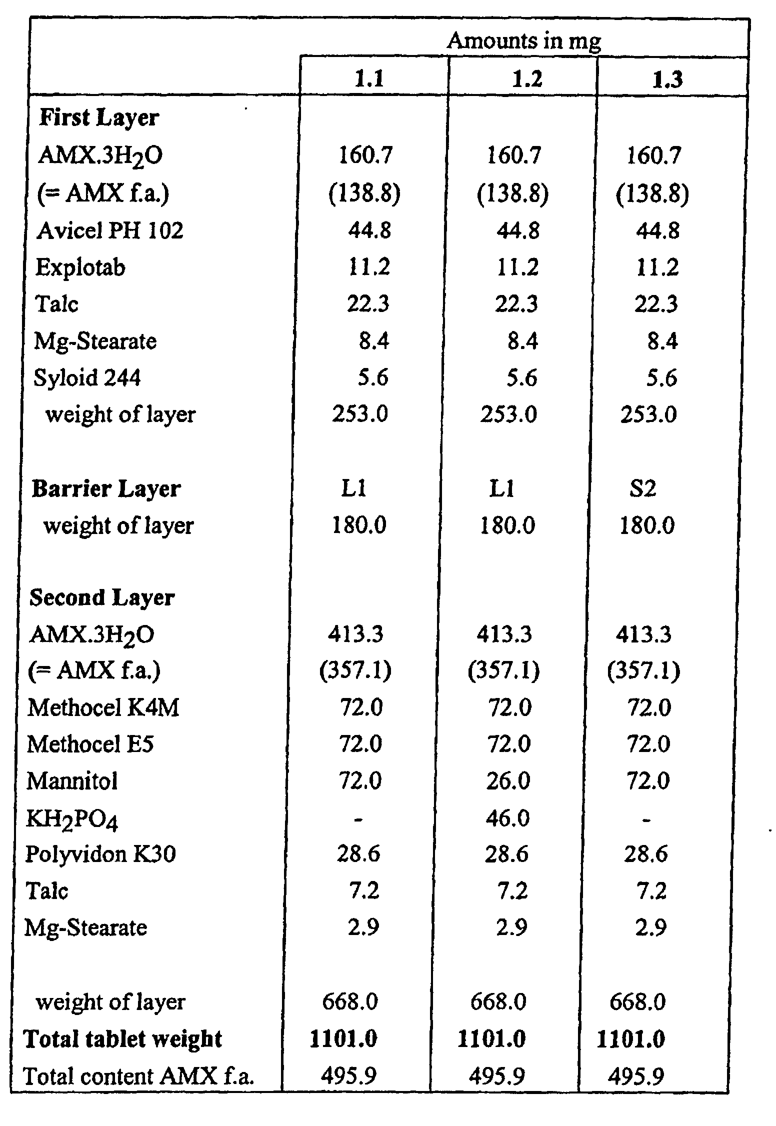

### Reference Example 2

Tablets 2.1 and 2.2 each having a structure as shown in Fig. 1B were prepared using an oval biconvex punch 21 x 10mm, having a first layer which is a rapid disintegrating layer, a second layer which is a slow-release layer, and a barrier layer sandwiched between them. These tablets had the following composition:

| | Amounts in mg | |
|---|---|---|
| **First Layer** | **2.1** | **2.2** |
| AMX.3H₂O | 118.7 | 118.7 |
| (= AMX f.a.) | (102.6) | (102.6) |
| AMX.3H₂O : KCA 1:1* | 83.4 | 83.4 |
| (= AMX f.a.) | (35.1) | (35.1) |
| (= CA f.a.) | (36.3) | (36.3) |
| Avicel PH 102 | 45.1 | 45.1 |
| Explotab | 11.4 | 11.4 |
| Talc | 22.4 | 22.4 |
| Mg-Stearate | 8.4 | 8.4 |
| Syloid 244 | 5.6 | 5.6 |
| | | |
| weight of layer | 295.0 | 295.0 |

| **Barrier Layer** | L1 | L1 |
|---|---|---|
| weight of layer | 160.0 | 160.0 |

| **Second Layer** | | |
|---|---|---|
| AMX.3H₂O | 305.27 | 305.27 |
| (= AMX f.a.) | (263.75) | (263.75) |
| AMX.3H₂O : KCA 1:1* | 214.40 | 214.40 |
| (= AMX f.a.) | (90.26) | (90.26) |
| (= CA f.a.) | (93.26) | (93.26) |
| Methocel K4M | 62.00 | 62.00 |
| Methocel E5 | 62.00 | 62.00 |
| Mannitol | 46.00 | - |
| KH₂PO₄ | - | 46.00 |
| Polyvidon K30 | 10.03 | 10.03 |
| Talc | 5.00 | 5.00 |
| Mg-Stearate | 20.30 | 20.30 |
| weight of layer | 725.00 | 725.00 |
| **Total tablet weight** | **1180.00** | **1180.00** |
| | | |
| Total content AMX f.a. | 491.7 | 491.7 |
| Total content CA f.a. | 129.6 | 129.6 |

| | | |
|---|---|---|
| * Note: Potassium clavulanate was used in the form of a commercially available 1 : 1 mixture of potassium clavulanate and amoxycillin trihydrate. | | |

### Reference Example 3

Tablets 3.1 and 3.2 each having a structure as shown in Fig 1C were prepared, using respectively oval biconvex punch 18 x 8 mm and 21 x 10 mm, having a first layer which is a slow release layer, a second layer which is also a slow release layer and a barrier layer located on the end face of the second layer. These tablets had the following composition:

| Amounts in mg | | |
|---|---|---|
| **First Layer** | **3.1** | **3.2** |
| AMX.3H₂O | 305.27 | 327.52 |
| (= AMX f.a.) | (263.75) | (282.98) |
| Methocel K4M | 53.18 | 57.06 |
| Methocel E5 | 53.18 | 57.06 |
| Mannitol | 53.18 | 57.06 |
| Polyvidon K30 | 18.03 | 19.34 |
| Talc | 5.31 | 5.70 |
| Mg-Stearate | 5.85 | 6.26 |
| | | |
| weight of layer | 494.00 | 530.00 |

| **Second Layer** | | |
|---|---|---|
| AMX.3H₂O : KCA 1:1 | 202.04 | 214.40 |
| (= AMX f.a.) | (85.06) | (90.26) |
| (= CA f.a.) | (87.89) | (93.26) |
| Methocel K4M | 35.20 | 37.35 |
| Methocel E5 | 35.20 | 37.35 |
| Mannitol | 35.20 | 37.35 |
| Polyvidon K30 | 11.99 | 12.72 |
| Talc | 3.52 | 3.73 |
| Mg-Stearate | 3.85 | 4.10 |
| | | |
| weight of layer | 327.00 | 347.00 |
| | | |
| **Barrier Layer** | S6 | S6 |
| weight of layer | 150.00 | 180.00 |
| | | |
| **Total tablet weight** | **971.00** | **1057.00** |
| | | |
| Total content AMX f.a. | 348.8 | 373.3 |
| Total content CA f.a. | 87.9 | 93.3 |

### Reference Example 4

Tablets 4.1, 4.2, 4.3, 4.4, 4.5 and 4.6, each having a structure as shown in Fig 1B were prepared, using oval biconvex punches 21 x 10 mm, having a first layer which is a slow-release layer, a second layer which is also a slow-release layer, and a barrier layer sandwiched between the first and second layers. These tablets had the following compositions.

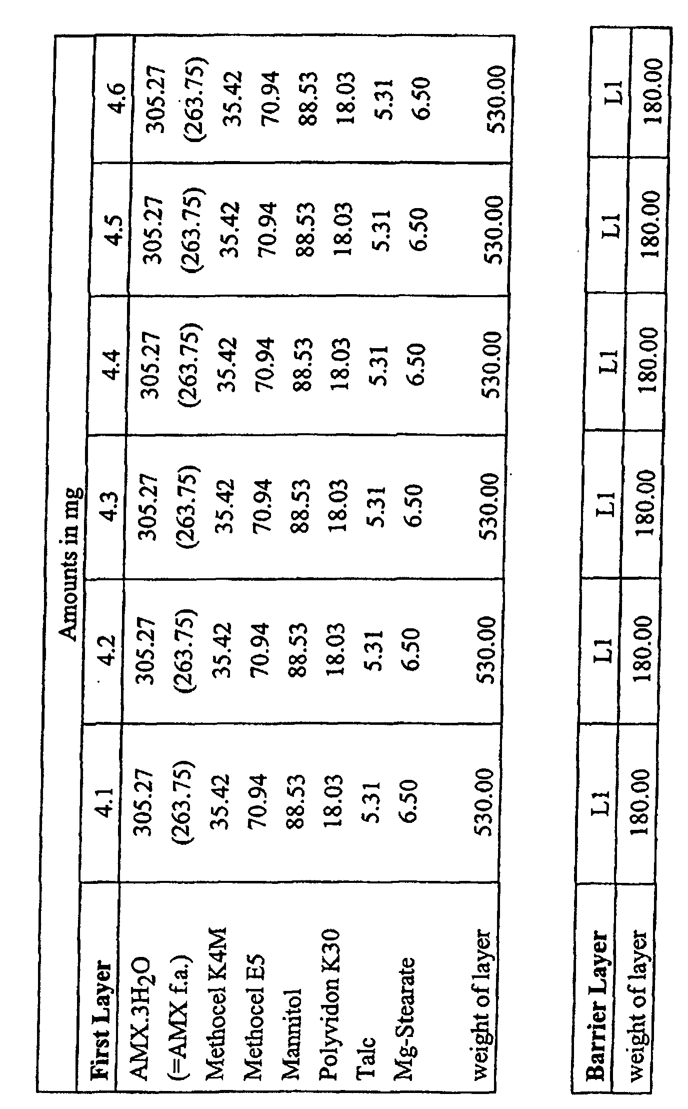

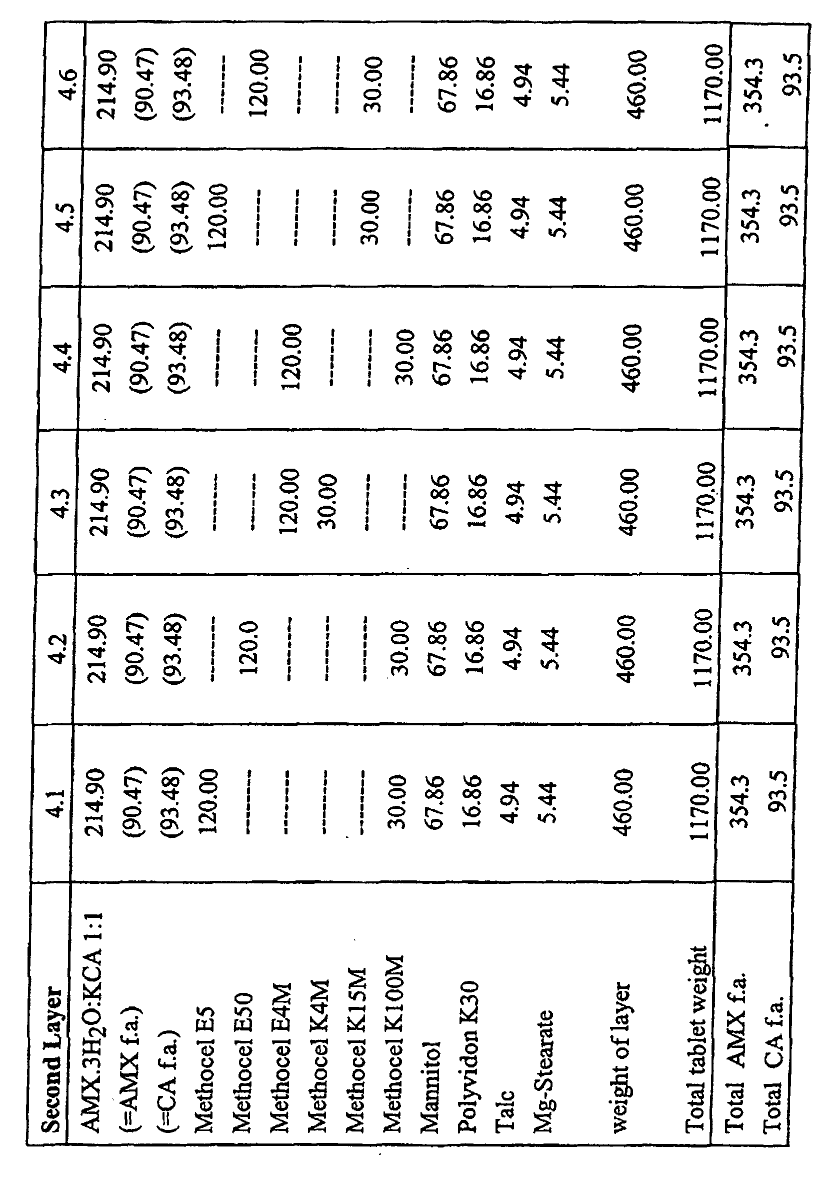

### Reference Example 5

Tablets 5.1, 5.2, 5.3, and 5.4 were prepared having a structure as shown in Fig. 1B.

| Amounts | | | | |
|---|---|---|---|---|
| **First Layer** | 5.1 | 5.2 | 5.3 | 5.4 |
| AMX.3H₂O(mg) | 320 | 215 | 320 | 210 |
| Methocel K4M% | 8.2 | 8.2 | 8.2 | 8.2 |
| Methocel E5% | 16.4 | 16.4 | 16.4 | 16.4 |

| **Barrier Layer** | L1 | L1 | L1 | L1 |
|---|---|---|---|---|
| **Second Layer** | | | | |
| AMX.3H₂O (mg) | 121 | 121 | 121 | 121 |
| KCA (mg) | 125 | 125 | 125 | 125 |
| Methocel K4M % | 10 | 30 | ----- | ----- |
| Methocel K100M % | ----- | ----- | 10 | 20 |

### Reference Example 6

Tablets 6.1 and 6.2 were prepared having a structure as shown in Fig. 1C.

| | Amounts in mg | |
|---|---|---|
| **First Layer** | **6.1** | **6.2** |
| AMX.3H₂O | 438.71 | 438.71 |
| (= AMX f.a.) | (379.05) | (379.50) |
| AMX.3H₂O/K-CA 1:1 | 103.45 | 103.45 |
| (= AMX f.a.) | (43.55) | (43.55) |
| (= CA f.a.) | (45.0) | (45.0) |
| Methocel K4M | 26.91 | 26.91 |
| Methocel E5 | 107.63 | 107.63 |
| Polyvidon K30 | 25.20 | 25.20 |
| Talc | 9.36 | 9.36 |
| Mg-Stearate | 8.64 | 8.64 |
| weight of layer 1 | 719.90 | 719.90 |

| **Second Layer** | | |
|---|---|---|
| AMX.3H₂O/K-CA 1:1 | 183.91 | 183.91 |
| (= AMX f.a.) | (77.4) | (77.4) |
| (CA f.a.) | (80.0) | (80.0) |
| Methocel K15M | 32.04 | ----- |
| Methocel K100M | ----- | 32.04 |
| Methocel E50 | 32.04 | 32.04 |
| Polyvidon K30 | 8.75 | 8.75 |
| Talc | 3.30 | 3.30 |
| Mg-Stearate | 3.05 | 3.05 |
| weight of layer 2 | 263.09 | 263.09 |
| **Barrier layer** | S6 | S6 |
| **weight of layer** | 180.00 | 180.00 |
| | | |
| **Total tablet weight** | **1162.99** | **1162.99** |

### Reference Example 7

Tablets 7.1, 7.2 and 7.3 were prepared having a structure as shown in Figs 1B (7.1) and 1E (7.2, 7.3).

| | Amounts in mg |
|---|---|
| **First Layer** | **7.1** |
| AMX.3H₂O | 438.71 |
| (= AMX f.a.) | (379.05) |
| AMX.3H₂O/K-CA 1:1 | 103.45 |
| (= AMX f.a.) | (43.55) |
| (= CA f.a.) | (45.00) |
| Methocel E5 | 134.64 |
| Polyvidon K30 | 25.20 |
| Talc | 9.36 |
| Mg-Stearate | 8.64 |
| weight of layer 1 | 720.00 |
| **Barrier layer** | L1 |
| **weight of layer** | 180.00 |
| **Second Layer** | |
| AMX.3H₂O/K-CA 1:1 | 183.91 |
| (= AMX f.a.) | (77.4) |
| (= CA f.a.) | (80.0) |
| Methocel K100M | 49.71 |
| Polyvidon K30 | 8.70 |
| Talc | 3.23 |
| Mg-Stearate | 2.98 |
| weight of layer 3 | 248.53 |
| **Total tablet weight** | **1148.53** |

In tablet 7.1 the potassium clavulanate in the first layer is a rapid release component, as the erodable polymer Methocel E5 allows rapid dissolution of soluble potassium clavulanate.

| | Amounts in mg | |
|---|---|---|
| **First Layer** | **7.2** | **7.3** |
| AMX.3H₂O | 319.96 | 319.96 |
| (= AMX f.a.) | (276.45) | (276.45) |
| Methocel E5 | 100.00 | 100.00 |
| Mannitol | 50.04 | 50.04 |
| Polyvidon K30 | 17.50 | 17.50 |
| Talc | 6.50 | 6.50 |
| Mg-Stearate | 6.00 | 6.00 |
| | | |
| weight of layer 1 | 500.00 | 500.00 |

| **Third Layer** | | |
|---|---|---|
| AMX.3H₂O/K-CA 1:1 | 203.91 | 203.91 |
| (=AMX f.a.) | (85.85) | (85.85) |
| (= CA f.a.) | (88.70) | (88.70) |
| Methocel K100M | 55.12 | 80.01 |
| CaHPO₄ | ----- | 92.08 |
| Polyvidon K30 | 9.65 | 14.01 |
| Talc | 3.58 | 5.20 |
| Mg-Stearate | 3.30 | 4.79 |
| weight of layer 2 | 275.56 | 400.00 |

| **Second Layer** | | |
|---|---|---|
| AMX.3H₂O | 118.7 | 118.7 |
| (= AMX f.a.) | (102.6) | (102.6) |
| AMX.3H₂O/K-CA 1:1 | 83.4 | 83.4 |
| (= AMX f.a.) | (35.1) | (35.1) |
| (= CA f.a.) | (36.3) | (36.3) |
| Avicel PH 102 | 45.1 | 45.1 |
| Explotab | 11.4 | 11.4 |
| Polyvidon K30 | 10.0 | 10.0 |
| Talc | 12.4 | 12.4 |
| Mg-Stearate | 8.4 | 8.4 |
| Syloid 244 | 5.6 | 5.6 |
| | | |
| weight of layer 3 | 295.0 | 295.0 |
| **Total tablet weight** | 1070.56 | 1195.00 |

In tablets 7.2 and 7.3 all three of the layers contain active material content, first and second layers being slow-release layers, and the intermediate third layer being a rapid-release layer.

### Reference Example 8

Tablets 8.1 and 8.2 were prepared having a structure as shown in Fig. I B.

| | Amounts in mg | |
|---|---|---|
| **First Layer** | **8.1** | **8.2** |
| AMX.3H₂O | 118.7 | 118.7 |
| (= AMX f.a.) | (102.6) | (102.6) |
| AMX.3H₂O/K-CA 1:1 | 83.4 | 83.4 |
| (= AMX f.a.) | (35.1) | (35.1) |
| (= CA fa.) | (36.3) | (36.3) |
| Avicel PH 102 | 45.1 | 45.1 |
| Explotab | 11.4 | 11.4 |
| Polyvidon K30 | 10.0 | 10.0 |
| Talc | 12.4 | 12.4 |
| Mg-Stearate | 8.4 | 8.4 |
| Syloid 244 | 5.6 | 5.6 |
| | | |
| weight of first layer | 295.0 | 295.0 |
| **Barrier layer** | S6 | RSB1 |
| **weight of layer** | 180.00 | 180.00 |

| **Second Layer** | | |
|---|---|---|
| AMX.3H₂O | 320.02 | 320.02 |
| (= AMX f.a) | (276.5) | (276.5) |
| AMX.3H₂O/K-CA 1:1 | (203.91) | 203.91 |
| (= AMX f.a.) | (85.8) | (85.8) |
| (=CA f.a.) | (88.7) | (88.7) |
| Methocel K4M | 62.00 | 62.00 |
| Methocel E5 | 62.00 | 62.00 |
| Mannitol | 31.74 | 31.74 |
| Polyvidon K30 | 20.03 | 20.03 |
| Talc | 5.00 | 5.00 |
| Mg-Stearate | 20.30 | 20.30 |
| | | |
| weight of second layer | 725.00 | 725.00 |
| Total tablet weight | 1200.00 | 1200.00 |

### Reference Example 9

Tablets 9.1 and 9.2 were prepared having a structure as shown in Figs 1B (9.1) and 1C (9.2).

| | Amounts in mg | |
|---|---|---|
| **First Layer** | **9.1** | **9.2** |
| AMX.3H₂O | 329.05 | 329.05 |
| (= AMX f.a.) | (248.3) | (248.3) |
| Methocel E5 | 102.84 | 102.84 |
| Mannitol | 51.46 | 51.46 |
| Polyvidon K30 | 18.00 | 18.00 |
| Talc | 6.68 | 6.68 |
| Mg-Stearate | 6.17 | 6.17 |
| weight of first layer | 514.20 | 514.20 |
| | **9.1** | **9.2** |
| **Barrier layer** | L1 | Second layer of 9.1 |
| **weight of layer 2** | 180.00 | 374.82 |

| **Second Layer** | **9.1** | **9.2** |
|---|---|---|
| AMX.3H₂O/K-CA 1:1 | 215.52 | Barrier L1 |
| (= AMX f.a.) | (90.73) | |
| (= CA f.a.) | (93.75) | |
| Methocel K4M | 74.96 | |
| Eudragit L* | 56.22 | |
| Dibutylphthalate* | 5.62 | |
| Polyvidon K30 | 13.12 | |
| Talc | 4.87 | |
| Mg-Stearate | 4.50 | |
| | | |
| weight of layer3 | 374.81 | |
| Total tablet weight | 1069.01 | 1069.01 |

| | | |
|---|---|---|
| * In these tablets the Eudragit L and plasticiser were coated directly onto the active material content by repeated steps of wet granulation. Two further examples of tablets (9.3 and 9.4) were prepared to the formula for 9.1 and 9.2 by first preparing a granulate of amoxycillin plus potassium clavulanate plus Methocel K4M, then coating the granulate with the Eudragit L. | | |

### Reference Example 10

Tablets 10.1 and 10.2 having a structure as shown in Fig. 1C were prepared

| | Amounts in mg | |
|---|---|---|
| **First Layer** | **10.1** | **10.2** |
| AMX.3H₂O | 438.68 | 438.68 |
| (= AMX f.a.) | (379.0) | (379.0) |
| Methocel E5 | 53.00 | 53.00 |
| Mannitol | 6.52 | 6.52 |
| Polyvidon K30 | 18.55 | 18.55 |
| Talc | 6.89 | 6.89 |
| Mg-Stearate | 6.36 | 6.36 |
| | | |
| weight of first layer 1 | 530.00 | 530.00 |

| **Second Layer** | | |
|---|---|---|
| AMX.3H₂O/K-CA 1:1 | 287.36 | 287.36 |
| (--AMxf.a.) | (121.0) | (121.0) |
| (= CA f.a.) | (125.0) | (125.0) |
| Methocel K4M | 34.50 | ----- |
| Methocel K100M | ----- | 34.50 |
| Mannitol | 2.44 | 2.44 |
| Polyvidon K30 | 12.08 | 12.08 |
| Talc | 4.48 | 4.48 |
| Mg-Stearate | 4.14 | 4.14 |
| | | |
| weight of layer 2 | 345.00 | 345.00 |
| **Barrier layer** | S6 | S6 |
| **weight of layer 2** | 180.00 | 180.00 |
| | | |
| **Total tablet weight** | 1055 | 1055 |

Two further examples of tablets (10.3 and 10.4) were prepared by coating tablets 10.1 and 10.2 with Eudragit L.

### Reference Example 11

Tablets 11.1 having a structure as shown in Fig. 1B were prepared

| | Amounts in mg |
|---|---|
| **First Layer** | **11.1** |
| AMX.3H₂O | 270.85 |
| (= AMX f.a.) | (234.0) |
| AMX.3H₂O/K-CA 1:1 | (206.90) |
| (= AMX f.a.) | (87.1) |
| (= CA f.a.) | (90.0) |
| Compritol 888 | 225.00 |
| Polyvidon K30 | 26.25 |
| Talc | 9.75 |
| Mg-Stearate | 11.25 |
| weight of first layer 1 | 750.00 |
| **Barrier layer** | S2 |
| **weight of layer 2** | 120 |
| **Second Layer** | |
| AMX.3H₂O | 167.86 |
| (= AMX f.a.) | (145.0) |
| AMX.3H₂O/K-CA 1:1 | 80.46 |
| (= AMX f.a.) | (33.9) |
| (= CA f.a.) | (35.0) |
| Avicel PH 102 | 53.21 |
| Explotab | 14.19 |
| Talc | 14.19 |
| Polyvidon K-30 | 12.42 |
| Syloid 244 | 7.09 |
| Mg-Stearate | 5.32 |
| | |
| weight of layer 3 | 345.74 |
| | |
| Total tablet weight | 1224.74 |

Potassium clavulanate was embedded in Compritol 888 by repeated steps of wet granulation. The first layer was a slow-release layer, and the second layer was a rapid-release layer.

### In Vitro and In Vivo Test Results

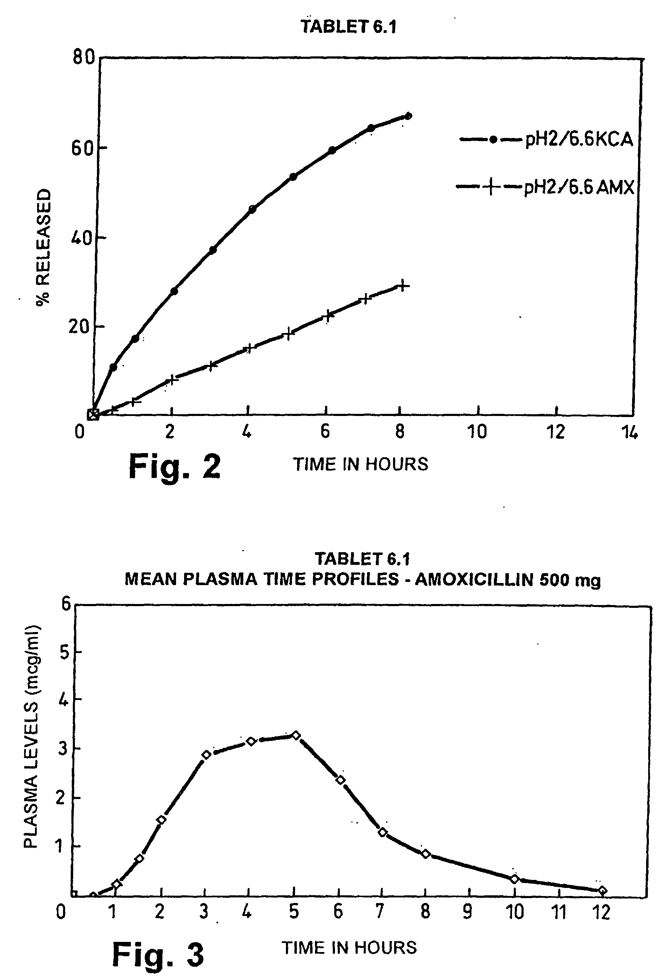

*In vitro* dissolution tests were carried out in a dual system, e.g. flowing media 2 hours at pH 2.2 followed by 6 hours at pH 6.6. Fig 2 shows results for tablet 6.1 from which amoxycillin and clavulanate were released with completion of release being almost reached within 8 hours.

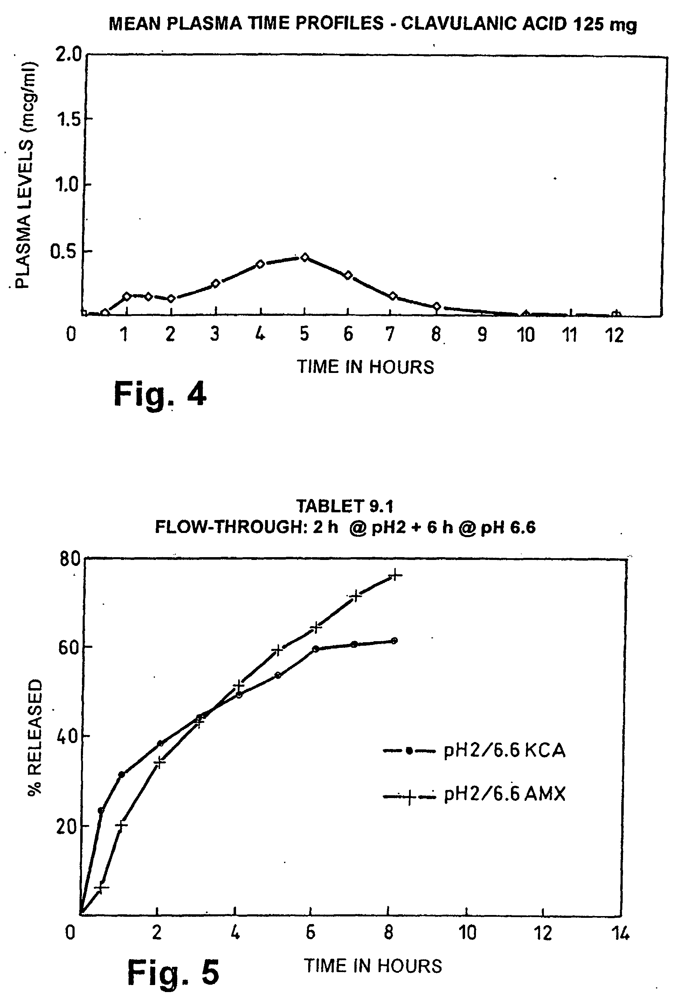

*In vivo* tests using tablet 6.1 were carried out on fed (milk) volunteers, and as shown in Fig 3 showed a prolonged amoxycillin plasma level. Similarly as. shown in Fig 4 a prolonged release of clavulanate was also experienced. The tablet 6.1 consequently shows a prolonged release profile of amoxycillin and clavulanate.

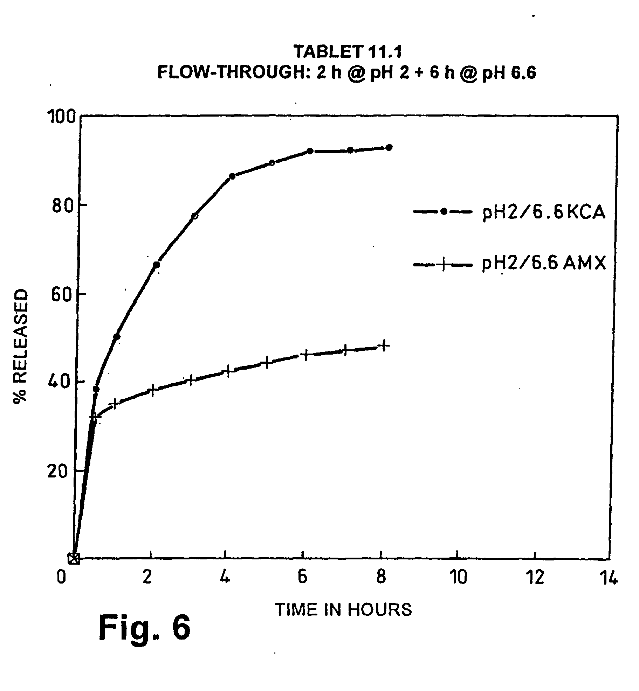

Fig.5 shows *in vitro* dissolution test results for tablet 9.1, and Fig.6 shows results for tablet 11.1, both showing prolongation of the release of clavulanate.

## Claims

1. A bilayer tablet formulation for oral administration, consisting of a first layer which includes amoxycillin and a second layer which includes amoxycillin wherein the relative rate of release of amoxycillin from the first and second layers differs and optionally a coating layer.

2. A tablet formulation according to claim 1 wherein the amoxycillin is in the form of amoxycillin trihydrate.

3. A tablet formulation according to claim 1 or 2 wherein differing rates of release are achieved by a first layer which is a rapid release layer which releases the bulk of its active material content within a relatively short time, and a second layer which is a slow release layer which releases the bulk of its active material content during a relatively long period after oral ingestion or a delayed release layer which releases the bulk of its active material content after a period of delay after oral ingestion, either in the stomach or in the intestine.

4. A tablet formulation according to claim 3 wherein the rapid release layer is a rapid disintegrating layer.

5. A tablet formulation according to claim 3 wherein the rapid release layer is a swellable layer having a composition which incorporates polymeric materials which swell rapidly and extensively in contact with water or aqueous media, to form a water permeable but relatively large swollen mass.

6. A tablet formulation according to any one of claims 3 to 5 wherein the slow release layer comprises a release retarding material which is a pH sensitive polymer; a release-retarding polymer which has a high degree of swelling in contact with water or aqueous media; a polymeric material which forms a gel on contact with water or aqueous media, or a polymeric material which has both swelling and gelling characteristics in contact with water or aqueous media.

7. A tablet formulation according to any one of the preceding claims wherein differing rates of release are achieved by having a first layer which is a slow or delayed release layer, and a second layer which is also a slow or delayed release layer.

8. A tablet formulation according to claim 7 wherein one or both of the first or second layers is a delayed release layer using enteric polymers.

9. A tablet formulation according to any one of the preceding claims in which the first layer is a rapid disintegrating layer and the second layer is a slow-release layer.

10. A method for the manufacture of a tablet formulation according to any one of the preceding claims comprising the steps of forming said first and second layers, and any coating layer(s) which may be present.

11. A tablet formulation according to any one of claims 1 to 15 for use as a therapeutic substance for oral administration for the treatment of bacterial infections.

## Patentansprüche

1. Zweischicht-Tablettenformulierung zur oralen Verabreichung, bestehend aus einer ersten Amoxycillin beinhaltenden Schicht und einer zweiten Amoxycillin beinhaltenden Schicht, wobei die relative Freisetzungsgeschwindigkeit von Amoxycillin aus der ersten und der zweiten Schicht unterschiedlich ist, und gegebenenfalls einer Überzugsschicht.

2. Tablettenformulierung nach Anspruch 1, wobei das Amoxycillin in Form von Amoxycillintrihydrat vorliegt.

3. Tablettenformulierung nach Anspruch 1 oder 2, wobei unterschiedliche Freisetzungsgeschwindigkeiten durch eine erste Schicht, bei der es sich um eine Schicht mit schneller Freisetzung handelt, die den Großteil ihres Wirkstoffgehalts innerhalb relativ kurzer Zeit freisetzt, und eine zweite Schicht, bei der es sich um eine Schicht mit langsamer Freisetzung, die den Großteil ihres Wirkstoffgehalts während eines relativ langen Zeitraums nach oraler Aufnahme freisetzt, oder eine Schicht mit verzögerter Freisetzung, die den Großteil ihres Wirkstoffgehalts nach einem Zeitraum der Verzögerung nach oraler Aufnahme, entweder im Magen oder im Darm, freisetzt, handelt, erzielt werden.

4. Tablettenformulierung nach Anspruch 3, wobei die Schicht mit schneller Freisetzung eine sich schnell zersetzende Schicht ist.

5. Tablettenformulierung nach Anspruch 3, wobei die Schicht mit schneller Freisetzung eine quellbare Schicht mit einer Zusammensetzung ist, die polymere Materialien beinhaltet, die bei Kontakt mit Wasser oder wäßrigen Medien schnell und ausgiebig quellen, um eine wasserdurchlässige, aber relativ große gequollene Masse zu bilden.

6. Tablettenformulierung nach einem der Ansprüche 3 bis 5, wobei die Schicht mit langsamer Freisetzung ein die Freisetzung verzögerndes Material umfaßt, bei welchem es sich um ein pH-empfindliches Polymer; ein die Freisetzung verzögerndes Polymer, das bei Kontakt mit Wasser oder wäßrigen Medien einen hohen Quellungsgrad aufweist; ein polymeres Material, das bei Kontakt mit Wasser oder wäßrigen Medien ein Gel bildet; oder ein polymeres Material, das bei Kontakt mit Wasser oder wäßrigen Medien sowohl Quell- als auch Gelbildungseigenschaften aufweist, handelt.

7. Tablettenformulierung nach einem der vorhergehenden Ansprüche, wobei unterschiedliche Freisetzungsgeschwindigkeiten dadurch erzielt werden, daß eine erste Schicht, bei der es sich um eine Schicht mit langsamer oder verzögerter Freisetzung handelt, und eine zweite Schicht, bei der es sich ebenfalls um eine Schicht mit langsamer oder verzögerter Freisetzung handelt, vorhanden sind.

8. Tablettenformulierung nach Anspruch 7, wobei es sich bei der ersten und/oder zweiten Schicht um eine Schicht mit verzögerter Freisetzung unter Verwendung von magensaftresistenten Polymeren handelt.

9. Tablettenformulierung nach einem der vorhergehenden Ansprüche, wobei es sich bei der ersten Schicht um eine sich schnell zersetzende Schicht und bei der zweiten Schicht um eine Schicht mit langsamer Freisetzung handelt.

10. Verfahren zur Herstellung einer Tablettenformulierung nach einem der vorhergehenden Ansprüche, umfassend die Schritte des Bildens der ersten und der zweiten Schicht und irgendwelcher möglicherweise vorhandenen Überzugsschichten.

11. Tablettenformulierung nach einem der Ansprüche 1 bis 15 zur Verwendung als Therapeutikum zur oralen Verabreichung zur Behandlung von bakteriellen Infektionen.

## Revendications

1. Formulation de tablettes bicouches pour l'administration par voie orale, consistant en une première couche qui comprend de l'amoxycilline et en une seconde couche qui comprend de l'amoxy-cilline, dans laquelle les vitesses relatives de libération de l'amoxycilline à partir de la première couche et à partir de la seconde couche different, et éventuellement en une couche d'enrobage.

2. Formulation de tablettes selon la revendication 1, dans laquelle l'amoxycilline est sous forme d'amoxycilline trihydrate.

3. Formulation de tablettes selon la revendication 1 ou la revendication 2, dans laquelle différentes vitesses de libération sont obtenues par une première couche qui est une couche à libération rapide qui libère le gros de son contenu de produit actif en un temps relativement court, et par une seconde couche qui est une couche à libération lente qui libère le gros de son contenu de produit actif sur une période de temps relativement longue après l'ingestion orale, ou par une couche à libération retardée qui libère le gros de son contenu en produit actif après un temps de retard consécutivement à l'ingestion orale, soit dans l'estomac, soit dans l'intestin.

4. Formulation selon la revendication 3, dans laquelle la couche à libération rapide est une couche à délitescence rapide.

5. Formulation selon la revendication 3, dans laquelle la couche à libération rapide est une couche déglutissable ayant une composition qui incorpore des matériaux polymères qui gonflent de manière rapide et extensive au contact de l'eau ou de milieux aqueux, pour former une masse gonflée perméable à l'eau mais relativement grosse.

6. Formulation de tablettes selon l'une quelconque des revendications 3 à 5, dans laquelle la couche à libération lente comprend un matériau retardateur de libération qui est un polymère sensible au pH ; un polymère retardateur de libération qui possède un degré élevé de gonflement au contact de l'eau ou de milieux aqueux ; un matériau polymère qui forme un gel au contact de l'eau ou de milieux aqueux, ou un matériau polymère qui a les caractéristiques à la fois de gonflemenyt et de gélification au contact de l'eau ou de milieux aqueux.

7. Formulation de tablettes selon l'une quelconque des revendications précédentes, dans laquelle différentes vitesse de libération sont obtenues en ayant une première couche qui est une couche à libération lente ou à libération retardée, et une seconde couche qui est aussi une couche à libération lente ou à libération retardée.

8. Formulation de tablettes selon la revendication 7, dans laquelle l'une ou les deux des première ou seconde couches sont des couches à libération retardée par l'emploi de polymères entériques.

9. Formulation de tablettes selon l'une quelconque des revendications précédentes, dans laquelle la première couche est une couche à délitescence rapide et la seconde couche est une couche à libération lente.

10. Technique de fabrication de tablettes d'une formulation selon l'une quelconque des revendications précédentes, comprenant les étapes de formation desdites première et seconde couches, et d'enrobage de toute couche susceptible d'être présente.

11. Formulation de tablettes selon l'une quelconque des revendications 1 à 15, en vue de l'utilisation en tant que substance thérapeutique pour l'administration orale dans le traitement d'infections bactériennes.
